(19) Europäisches Patentamt
European Patent Office
Office européen des brevets

(11) Veröffentlichungsnummer: **0 276 702 B1**

# (12) EUROPÄISCHE PATENTSCHRIFT

(45) Veröffentlichungstag der Patentschrift: **05.12.90**

(51) Int. Cl.[5]: **C13K 13/00**

(21) Anmeldenummer: **88100515.1**

(22) Anmeldetag: **15.01.88**

## (54) Verfahren zur Herstellung von Kristalliner L-Arabinose.

(30) Priorität: **29.01.87 DE 3702653**

(43) Veröffentlichungstag der Anmeldung: **03.08.88 Patentblatt 88/31**

(45) Bekanntmachung des Hinweises auf die Patenterteilung: **05.12.90 Patentblatt 90/49**

(84) Benannte Vertragsstaaten: **AT BE CH DE FR GB IT LI NL SE**

(56) Entgegenhaltungen:
**GB-A- 1 182 099**

**CHEMICAL ABSTRACTS, Band 71, Nr. 9, 3. November 1969, Seiten 118-119, Zusammenfassung Nr. 82869t, Columbus, Ohio, US; & CS-A-129 664 (V. TIBENSKY) 15-04-1968**
**CHEMICAL ABSTRACTS, Band 89, Nr. 12, September 1978, Seite 73, Zusammenfassung Nr. 91345t, Columbus, Ohio, US; & JP-A-78 59 699 (SANYO-KOKUSAKU PULP CO., LTD) 29-05-1978**
**CHEMICAL ABSTRACTS, Band 93, Nr. 15, Oktober 1980, Seite 752, Zusammenfassung Nr. 150586e, Columbus, Ohio, US; & CS-A-181 485 (A. KRAMAR et al.) 15-01-1980**

(73) Patentinhaber: **Süddeutsche Zucker-Aktiengesellschaft, Maximilianstrasse 10, D-6800 Mannheim 1(DE)**

(72) Erfinder: **Schiweck, Hubert, Dr., John-F.-Kennedy-Strasse 5, D-6520 Worms(DE)**
Erfinder: **Vogel, Manfred, Dr., Beim Bergtor 28, D-6718 Grünstadt(DE)**

(74) Vertreter: **Körber, Wolfhart, Dr.rer.nat. et al, Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10, D-8000 München 22(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur Herstellung von L-Arabinose aus einem arabanhaltigen Pflanzenmaterial, besonders Rübenaraban, welches aus entzuckerten Rübenschnitzeln extrahiert wird.

Araban kommt in vielen Pflanzengummis, wie Gummi arabicum, Kirschgummi, aber auch als Bestandteil des Rübenmarks vor. Das in der Natur weit verbreitete Araban liegt dort immer mit pektinhaltigen Substanzen vergesellschaftet vor.

Das Arabanmolekül besitzt ein relativ niedriges Molekulargewicht und weist neben linear α-1,5-verknüpften L-Arabofuranose-Einheiten auch α-1,3-Verknüpfungen auf; demzufolge ist es ein stark verzweigtes Polysaccharid. Araban ist indessen dein homopolymeres Molekül, welches nur aus L-Arabinose aufgebaut ist. So lassen sich im Rübenaraban nach sauerer Hydrolyse u.a. D-Galaktose, L-Rhamnose und D-Galakturonsäure nachweisen.

Eine Möglichkeit, L-Arabinose aus Pflanzenmaterial zu gewinnen, besteht in der saueren Hydrolyse des Ausgangsmaterials, gefolgt von der Isolierung der L-Arabinose aus dieser Lösung. Solche Versuche wurden von S. Harding (Sugar 24, 656, 1922) zusammengestellt. E.Anderson und L. Sands (Org. Synth. 1, 67, 1941) beschreiben die Herstellung von L-Arabinose bei der Verwendung von Mesquite-Gummi als Ausgangsmaterial. Die Produktlösung enthält neben L-Arabinose noch größere Verunreinigungen an anderen Kohlenhydraten, wodurch aufwendige Reinigungsschritte notwendig werden.

In den CS-PSen 129 664 und 137 537 wird daher nach Neutralisation des saueren Hydrolysates eine Hefe-Fermentation vor genommen, um störende Kohlenhydrate zu entfernen. Anschließend werden hochmolekulare Verunreinigungen durch Ethanol gefällt und die L-Arabinose aus ethanolischer Lösung kristallisiert.

Neben der saueren Hydrolyse ist auch die Extraktion des Arabans im Alkalischen bereits beschrieben worden, so von A.E. Goodban und H.S. Owens (J. Am. Soc. Sugar Beet Technol. 9, 129 - 132, 1956). Sie konnten zeigen, daß das Araban aus den Gerüstsubstanzen bei 100°C und pH 11 nach 40 Minuten nahezu quantitativ in Lösung geht. In Gegenwar t einer ausreichenden Menge Kalkmilch lassen sich nach zweistündiger Aufenthaltszeit bei gleicher Temperatur ähnliche Resultate erzielen.

Nach der DD-PS 143 261 werden Zuckerrübenschnitzel zuerst intensiv mit Wasser gewaschen, um die restliche Saccharose zu entfernen; die Extraktion des Arabans erfolgt dann bei einer Temperatur zwischen 80 bis 100°C, vorzugsweise bei 95°C und einer Verweilzeit von 10 bis 120 Minuten, vorzugsweise 30 Minuten, mit $Ca(OH)_2$-Lösungen.

In der EP-A-0115 068 wird eine selektive Adsorption von L-Arabinose an Zeolithe als Reinigungsschritt beschrieben. Andere Zucker sollen nicht gebunden werden. Die Elution der L-Arabinose erfolgt mit Wasser oder wässerigen Mischungen von Alkoholen oder Ketonen.

Es wurde nun überraschenderweise gefunden, daß L-Arabinose aus rein wäßrigen Systemen kristallin in guter Ausbeute gewonnen werden kann, wenn zuerst das Araban aus den entzuckerten Rübenschnitzeln bei erhöhter Temperatur unter Druck im Alkalischen aus der Reaktionslösung herausgelöst wird. Das Inlösunggehen des Arabans erfolgt dabei schnell und nahezu quantititav. Im Gegensatz zu anderen Verfahren ist eine Vorbehandlung des Pflanzenmaterials, wie ausgiebiges Waschen, nicht notwendig.

Die Konzentrationen an $Ca(OH)_2$ in der Reaktionslösung sind mit 0,5 bis 2,0 Gew.-% bzw. einem Verhältnis von 6 bis 17 Gew.-% $Ca(OH)_2$, pro kg Trockensubstanz bei einer Verweilzeit von 2 bis 20 Minuten und einer Temperatur von 105 bis 160°C, entsprechend einem Druck von 1,5 bis 7 bar, so gewählt, daß der größte Anteil des Pektins zerstört wird. Dies hat zur Folge, daß die Viskosität der Reaktionslösung verringert wird und sie sich leicht filtrieren läßt. Der Druck liegt dabei höher als er dem Sattdampfdruck entspricht.

Nach dem Abkühlen auf z.B. 40 bis 60°C wird die alkalische Reaktionslösung unter Rühren durch Zugabe einer Säure, besonders Schwefelsäure, neutralisiert; die ungelösten pflanzlichen Rückstände werden dann zusammen mit dem anorganischen Niederschlag durch Filtration von der arabanhaltigen Flüssigkeit abgetrennt.

Die so erhaltene Arabanlösung wird nun auf einen Trockensubstanzgehalt von 40 bis 60 % eingedampft und ggf. nochmals filtriert.

Die Erfindung betrifft weiter die Isolierung der L-Arabinose auf chromatographischem Wege. Zunächst wird die Arabanlösung über einen Ca-beladenen Kationenaustauscher in Fraktionen getrennt. Dies erfolgt zweckmäßig bei Temperaturen zwischen 70 bis 80°C. Dabei wird unter ähnlichen Bedingungen getrennt, wie sie z.B. von der Trennung von Glucose und Fruktose ausgehend von Invertzucker bekannt sind. Wie Abbildung 1 zeigt, wird dabei das Araban zuerst eluiert, gefolgt von Saccharose und ionischen Substanzen. Die Saccharose stammt von den extrahierten Rübenschnitzeln, bei den Salzen handelt es sich um die Ca-Salze von organischen Säuren, die beim Abbau des Pektins entstehen.

Die arabanhaltigen Fraktionen, z.B. 0,5 Bettvolumina, werden durch Zugabe von $H_2SO_4$ hydrolysiert; dazu wird die Lösung auf 0,5 bis 2, besonders 1 Gew.-% dieser Säure gebracht. Die Hydrolyse erfolgt bei einer Temperatur von 92 bis 97°C während 50 bis 80 Minuten, vorzugsweise bei 93 bis 95°C während 70 Minuten. Unter diesen Bedingungen wird die L-Arabinose nahezu vollständig aus dem Araban gelöst und die störenden Kohlenhydrate, vornehmlich D-Galaktose, L-Rhamnose und D-Galakturonsäure verbleiben in oligomerer / polymerer Form.

Die Neutralisation obiger Hydrolyselösung erfolgt durch Zugabe von $CaCO_3$, der entstandene Niederschlag wird abfiltriert. Die Lösung wird auf 40 bis 60 % Trockensubstanzgehalt eingedampft, filtriert und am Ca-beladenen Kationenaustauscher bei erhöhter Temperatur, Z.B. 70 bis 80°C mit Wasser getrennt. Wie der Abbildung 2 zu entnehmen ist, werden entstandene farbige Verunreinigungen mit den polymeren Kohlenhydraten zuerst eluiert, gefolgt von der L-Arabinose, die eine Reinheit von 85 bis 89 % besitzt.

Durch das erfindungsgemäße Verfahren wird eine so hohe Reinheit in den L-arabinosehaltigen Fraktionen erreicht, daß die L-Arabinose im nachfolgenden Verfahrensschritt aus Wasser auskristallisiert werden kann.

Nach Vereinen und Einengen der L-arabinosereichen Fraktionen auf einen Trockensubstangehalt von 60 bis 80 %, besonders um 65 %, erfolgt die Kristallisation durch Kühlungskristallisation, indem die Lösung von z.B. 65°C auf 25°C abgekühlt wird. Es wird so eine L-Arabinose mit einer Reinheit von 94 bis 97% erhalten. Eine Umkristallisation aus Wasser ergibt eine Reinheit von > 98 %.

Die Kristallisation erfolgt in der Regel mehrstufig, wobei z.B. der Endsirup nach der dritten Kristallisation zur zweiten Chromatographie am Ca-beladenen Kationenaustauscher zurückgegeben wird.

Als Kationenaustauscher kommen in Betracht solche, deren Grundgerüst aus Polystyrolsulfonsäure besteht, die mit 3 bis 6 % Divinylbenzol vernetzt in der Ca-Form vorliegen, wie z.B. Lewatit® TSW 40 der Fa. Bayer oder Duolite® C'204 F der Fa. Rohm & Haas.

Die Erfindung wird durch die folgenden Beispiele näher erläutert:

Beispiel 1:

150 kg Preßschnitzel (27 % Trockensubstanzgehalt) werden mit 300 1 Wasser in Gegenwart von 5,3 kg $Ca(OH)_2$ unter Rühren auf 140°C schnell erhitzt (10 Minuten) und für 5 Minuten bei dieser Temperatur belassen. Der abgekühlte Brei wird mit 33 %iger $H_2SO_4$ unter Rühren neutralisiert. Nach Abtrennung des verbliebenen Feststoffes und einem Auswaschen desselben mit 100 1 Wasser, werden die Lösungen vereinigt und von5 % auf 45 % Trockensubstanzgehalt eingeengt. Es folgt eine Filtration bevor der Sirup bei 80°C an einer 500-1-Trennanlage, die mit einem schwach vernetzten Ca-beladenen Kationenaustauscher (etwa vom Typ TSW 40, Feinkorn, Fa. Bayer AG) gefüllt ist, mit Wasser getrennt wird. Die Elution erfolgt mit 115 1 Wasser/h.

Die arabanhaltigen Fraktionen werden vereint und mit 33 %iger $H_2SO_4$ (ca. 12 kg) auf 1 Gew.-% dieser Säure gebracht. Die Hydrolyse wird bei 93°C für 70 Minuten durchgeführt. Die Neutralisation erfolgt dann durch Zugabe von ca. 5 kg. $CaCO_3$, anschließend wird filtriert.

Die auf 45 % TS-Gehalt eingeengte und filtrierte Lösung wird an einer 330-1-Trennanlage, gefüllt mit Ca-beladenem Kationenaustauscher unter identischen Bedingungen rechromatographiert.

Die L-arabinosehaltigen Fraktionen werden vereint und auf 65 % Trockensubstanzgehalt bei 65°C eingeengt und im Kristallisator einer mehrstufigen Kühlungskristallisation unterzogen. Die Abkühlung erfolgt um 3°C pro Stunde. Die erhaltenen Kristalle werden abgenutscht. Die Reinheit beträgt 96 % bei einer Ausbeute von 2,7 kg.

Beispiel 2:

4,6 kg Trockenschnitzel (93 % Trockensubstanzgehalt) werden in einem Autoklav zu 35 1 95°C heißem Wasser gegeben, dem zuvor 600 g $Ca(OH)_2$ zugesetzt waren. Nach schnellem Aufheizen auf 140°C unter Rühren wird für 5 Minuten bei dieser Temperatur belassen.

Weitere Aufarbeitungsschritte wie in Beispiel 1.

Die Ausbeute an L-Arabinose betragt 260 g.

Beispiel 3:

200 kg Preßschnitzel (27 % Trockensubstanzgehalt) werden unter gleichzeitiger Zudosierung von 120 1 einer wäßrigen $Ca(OH)_2$-Suspension, die insgesamt 6 kg $Ca(OH)_2$ enthält, in Sekundenschnelle auf 140°C gebracht und 4 Minuten bei dieser Temperatur gehalten. Der abgekühlte Brei wird durch Zugabe von 33 %iger $H_2SO_4$ neutralisiert und die arabanhaltige Flüssigkeit über eine Membranfilterpresse abgetrennt. Mit dem zweifachen Volumen des Filterkuchens wird ausgewaschen.

Weitere Bearbeitungsschritte wie in Beispiel 1.

Die Ausbeute an L-Arabinose beträgt 3,9 kg.

## Patentansprüche

1.Verfahren zur Herstellung von Kristalliner L-Arabinose aus einem arabanhaltigen Pflanzenmaterial durch Aufschluß in einer $Ca(OH)_2$-haltigen Suspension, dadurch gekennzeichnet, daß man

a) das Araban bei Temperaturen zwischen 105 und 160°C bei dem sich einstellenden Druck in einem geschlossenen Gefäß während einer Reaktionszeit von 2 bis 20 Minuten unter Verwendung einer wässerigen Reaktionslösung mit einem Gehalt von 0,5 bis 2 Gew.% $Ca(OH)_2$ entsprechend einem Verhältnis von 6 bis 17 Gew.% $Ca(OH)_2$ pro kg Trockensubstanz in Lösung bringt.

b) die Reaktionslösung nach dem Abkühlen mit einer Säure neutralisiert und von dem unaufgeschlossenen Pflanzenmaterial und dem ausgefallenen anorganischen Niederschlag abfiltriert,

c) die erhaltene wäßrige Phase auf einen Trockensubstanzgehalt von 40 bis 60 % eindampft und anschließend über einen stark saueren, insbesonders schwach vernetzten Kationenaustauscher in der Ca-Form in eine arabanhaltige und nebenproduktkhaltige Fraktion trennt.

d) die arabanhaltige Fraktion mit einer 0,5 bis 2 gewichtsprozentigen wäßrigen $H_2SO_4$-Lösung bei 92 bis 97°C während 50 bis 80 Minuten hydrolysiert,

e) die Hydrolyselösung nach d) durch Zugabe von $CaCO_3$ neutralisiert, von Niederschlag abfiltriert und auf einen Trockensubstanzgehalt von 40 bis 60 % eindampft,

f) die eingeengte Lösung nach e) über einen stark saueren, insbensonders schwach vernetzten Kationenaustauscher in der Ca-Form in eine L-arabinosehaltige und nebenprodukthaltige Fraktion trennt,

g) die arabinosehaltige Fraktion nach Einengen auf Trockensubstanzgehalte von 60 bis 80 % einer Kühlungskristallisation unterwirft und die entstehenden Kristalle abtrennt, sowie ggf. die Mutterlauge rekristallisiert und die letzte Mutterlauge zur Trennung nach d) zurückführt.

2. Verfahren nach Anspruch 1, dadurch gekennzeichnet, daß der Aufschluß des arabanhaltigen Materials in einem Temperaturbereich von 130 bis 140°C mit einer mittleren Verweilzeit von 5 bis 10 Minuten erfolgt.

3. Verfahren nach Anspruch 1 und 2, dadurch gekennzeichnet, daß die Reaktionslösung 1 bis 1,5 Gew.-% an $Ca(OH)_2$ bzw. 10 lbis 15 Gew.-% $Ca(OH)_2$ pro kg Trockensubstanz enthält.

4. Verfahren nach Anspruch 1 bis 3, dadurch gekennzeichnet, daß die arabanhaltige Fraktion in einer 0,8 bis 1,2 gewichtsprozentigen wäßrigen $H_2SO_4$-Lösung bei 93 bis 95°C für 70 Minuten hydrolysiert wird.

## Claims

1. A process for preparing crystalline L-arabinose from an araban-containing plant material by hydrolysis in a $Ca(OH)_2$-containing suspension, characterised in that

a) the araban is brought into solution at temperatures between 105° and 160°C at the pressure developed in a closed vessel during a reaction time of 2 to 20 minutes using an aqueous reaction solution containing from 0.5 to 2% by weight of $Ca(OH)_2$ corresponding to a proportion of 6 to 17% by weight of $Ca(OH)_2$ per kilogramme of dry material,

b) after cooling the reaction solution is neutralised with acid and filtered from the unhydrolysed plant material and the inorganic precipitate formed,

c) the resulting aqueous phase is evaporated down to a dry substance content of from 40 to 60% and is then separated by means of a strongly acidic cation exchanger, in particular one that is slightly crosslinked, in the Ca form into an araban-containing fraction and fractions containing by-products,

d) the araban-containing fraction is hydrolysed with an aqueous 0.5 to 2% by weight $H_2SO_4$ solution at 92 to 97°C for 50 to 80 minutes,

e) the hydrolysis solution from d) is neutralised by addition of $CaCO_3$, filtered off from the precipitate

and evaporated down to a dry substance content of 40 to 60%,

f) the concentrated solution obtained by step e) is separated by means of a strongly acid cation exchanger, in particular one that is slightly crosslinked, in the Ca form into an L-arabinose-containing fraction and fractions containing by-products,

g) the arabinose-containing fraction, after concentration to a dry substance content of 60 to 80%, is subjected to crystallisation by cooling and the resulting crystals are separated off, and if desired the mother liquor is re-crystallised and the last mother liquor is returned to the separation according to d).

2. A process according to claim 1, characterised in that the hydrolysis of the araban-containing material takes place in a temperature range of 130 to 140°C with a mean residence time of 5 to 10 minutes.

3. A process according to claim 1 and claim 2, characterised in that the reaction solution contains 1 to 1.5% by weight of $Ca(OH)_2$ or 10 to 15% by weight $Ca(OH)_2$ per kilogramme dry substance as the case may be.

4. A process according to any one of claims 1 to 3, characterised in that the araban-containing fraction is hydrolysed in a 0.8 to 1.2% by weight aqueous $H_2SO_4$ solution at 93 to 95°C for 70 minutes.

## Revendications

1. Procédé de préparation de L-arabinose cristallin à partir d'une matière végétale contenant de l'arabane, par décomposition dans une suspension contenant du $Ca(OH)_2$, caractérisé en ce que:

a) l'arabane est mis en solution, à des températures entre 105 et 160°C à la pression qui s'établit dans un récipient fermé pendant un temps de réaction de 2 à 20 minutes, en utilisant une solution réactionnelle aqueuse avec une teneur de 0,5 à 2% en poids de $Ca(OH)_2$ selon une proportion de 6 à 17% en poids de $Ca(OH)_2$ par kg de matière sèche,

b) après refroidissement la solution de réaction est neutralisée ave un acide, et elle est séparée par filtration de la matière végétale non décomposée et du précipité inorganique déposé,

c) la phase aqueuse obtenue est concentrée par évaporation, jusqu'à une teneur en matière sèche de 40 à 60%, et elle est séparée ensuite sur un échangeur de cations fortement acide, en particulier faiblement réticulé dans la forme Ca, en une fraction contenant de l'arabane et une fraction contenant les sous-produits,

d) la fraction contenant l'arabane est hydrolisée avec une solution aqueuse de $H_2SO_4$ à 0,5 à 2% en poids, à 92 à 97°C pendant 50 à 80 minutes,

e) la solution d'hydrolyse selon d) est neutralisée par addition de $CaCO_3$, et elle est séparée du précipité par filtration et concentrée par évaporation jusqu'à une teneur en matière sèche de 40 à 60%,

f) la solution concentrée selon e) est séparée sur un échangeur de cations fortement acide, en particulier faiblement réticulé sous le forme Ca, en une fraction contenant de l'arabane et une fraction contenant les sous-produits,

g) après concentration jusqu'à une teneur en matière sèche de 60 à 80%, la fraction contenant de l'arabane est soumise à une cristallisation par refroidissement et les cristaux formés sont séparés, et aussi, le cas échéant, l'eau mère est recristallisée et la dernière eau-mère est recyclée vers la séparation selon d).

2. Procédé selon la revendication 1, caractérisé en ce que la décomposition de la matière contenant de l'arabane a lieu dans un domaine de température de 130 à 140°C, avec un temps de séjour moyen de 5 à 10 minutes.

3. Procédé selon les revendications 1 et 2, caractérisé en ce que la solution de réaction contient 1 à 1,5% en poids de $Ca(OH)_2$ ou bien 10 à 15% en poids de $Ca(OH)_2$ par kg de matière sèche.

4. Procédé selon les revendications 1 à 3, caractérisé en ce que la fraction contenant de l'arabane est hydrolysée dans une solution aqueuse de $H_2SO_4$ à 0,8 à 1,2% en poids, à une température de 93 à 95°C pendant 70 minutes.

Abb. 1

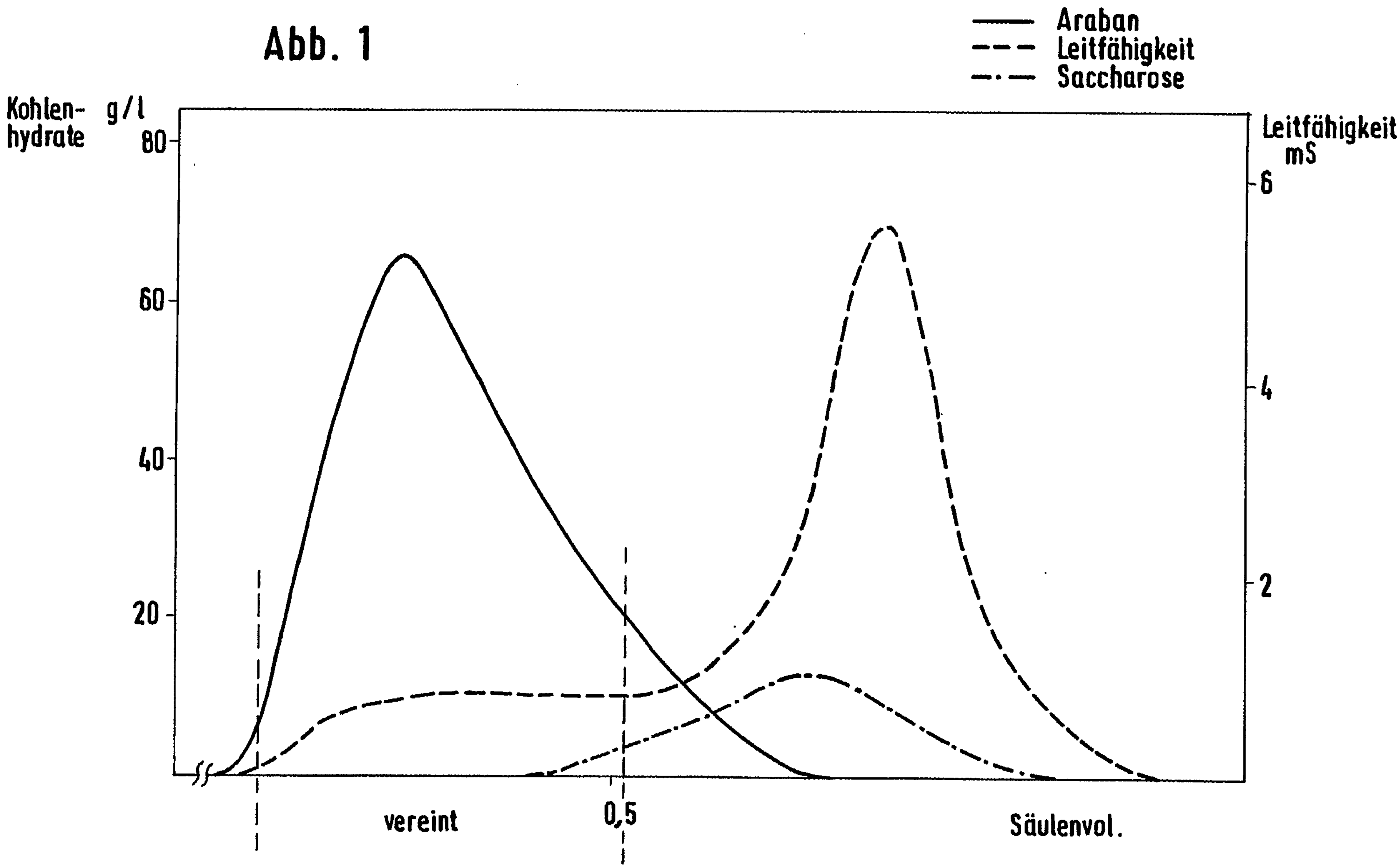
Araban
Leitfähigkeit
Saccharose
Kohlen-hydrate g/l
80
60
40
20
Leitfähigkeit mS
6
4
2
vereint
0,5
Säulenvol.

Abb. 2

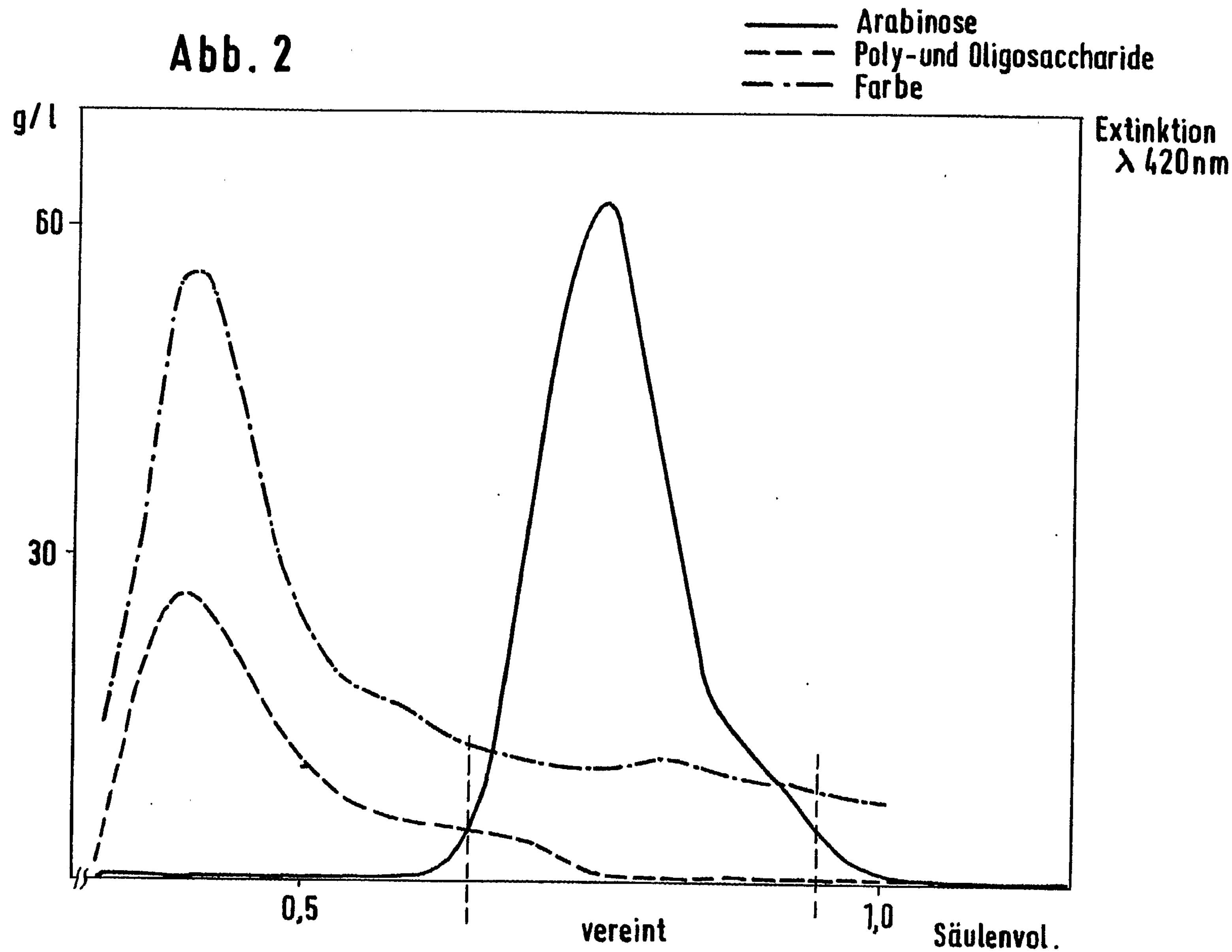
Arabinose
Poly-und Oligosaccharide
Farbe
g/l
Extinktion
λ 420nm
60
30
0,5
vereint
1,0
Säulenvol.